# EUROPEAN PATENT SPECIFICATION

(11) **EP 3 410 841 B1**
(45) Date of publication and mention of the grant of the patent: **10.04.2024**
(21) Application number: 17704077.1
(22) Date of filing: 07.02.2017
(51) Int. Cl.: A01G 31/02

(54) **FOG GENERATOR FOR AEROPONICS**
NEBELGENERATOR FÜR AEROPONISCHE KULTIVIERUNG
GÉNÉRATEUR DE BROUILLARD POUR LA CULTURE AEROPONIQUE

(30) Priority: 07.02.2016 US 201662292323 P; 01.04.2016 GB 201605610
(43) Date of publication of application: 12.12.2018
(73) Proprietor: Airponix Ltd, London, Surrey TW10 6TS (GB)
(72) Inventor: RUGGIER, Michael, London Surrey TW10 6TS (GB); PREWER, John, London Surrey TW10 6TS (GB)
(74) Representative: J A Kemp LLP
(86) International application number: PCT/GB2017/050299
(87) International publication number: WO 2017/134472

(56) References cited:
- BR-A- PI0 503 660
- CN-A- 103 621 393
- CN-B- 103 621 393
- US-A- 5 136 804
- US-A- 5 136 804
- US-A1- 2004 167 028
- US-A1- 2009 293 357
- US-A1- 2009 293 357
- US-A1- 2011 023 359
- US-A1- 2011 023 359
- US-A1- 2013 047 298
- US-A1- 2014 000 162
- US-A1- 2014 000 162
- US-A1- 2015 136 867
- S EDWARD LAW: "Agricultural electrostatic spray application: a review of significant research and development during the 20th century", JOURNAL OF ELECTROSTATICS (ELSEVIER), vol. 51/52, 1 January 2001 (2001-01-01), pages 25-42, XP002798621,

## Description

The present invention relates to an aeroponic propagator for the cultivation of plants and a method of supplying fog within an aeroponic propagator.

Aeroponics is a development of hydroponic methods. Hydroponics is the technique of growing plants in water-based solutions of nutrient salts. Although known over 100 years ago it was not used extensively until the Second World War, when it was used to provide troops with green vegetables in parts of the world where normal methods of cultivation were impractical. Hydroponic technology has since been matured and is widely used in many countries and has proved to be the most economical production method in greenhouses. Still initial costs are substantial, and rockwool/glasswool substrates cause vectors for spreading diseases, and create large heaps of incombustible waste after each crop and a large recurring cost. These drawbacks feed a continuous search for better technology.

A hydroponic system known as the nutrient film technique (NTT) was developed during the 1960s at the UK's Glasshouse Crops Research Institute by Dr. Alan Cooper. Although widely acclaimed as a significant advance in hydroponic growth techniques it has a number of drawbacks. The main ones being that - though simple in concept - it tends to be expensive to install and often has been difficult to operate profitably because of disease and nutrient control problems. In spite of these limitations, NTT's appeal to growers is such that it has been used in more than 70 countries.

Aeroponics has gained much publicity over recent years. It is defined by the International Society for Soil-less Culture as "A system where roots are continuously or discontinuously in an environment saturated with fine drops (a mist or aerosol) of nutrient solution". The method requires no substrate and entails growing plants with their roots suspended in a chamber (the root chamber), with the roots periodically atomised with a fine mist or fog of nutrients, a process which uses significantly less water than alternative growing techniques. Since their inception some 30 years ago, aeroponic techniques have proved very successful for propagation and are widely used in laboratory studies of plant physiology, but have yet to prove themselves on a commercial scale. Aeroponics could also have applications in crisis situations because an aeroponics system can be designed to work in the event of such things as reduced solar radiation levels (e.g. due to high levels of fine volcanic ash particles in the atmosphere) or floods.

US 2011/023359, US 2009/293357, US 5 136 804, CN 103 621 393 and US 2014/000162 disclose background art.

However, the main limitations associated with commercial aeroponic systems are high equipment costs, infrastructure costs (e.g. greenhouses) and low equipment reliability.

According to one aspect of the present invention there is provided an aeroponic propagator for the cultivation of plants, the aeroponic propagator comprising: a fogging system for supplying fog within the aeroponic propagator, wherein the fogging system comprises a droplet generator, the droplet generator comprising at least one outlet, and the droplet generator being configured such that in use each at least one outlet ejects one droplet of liquid at a time.

The aeroponic propagator may comprise multiple outlets, and the outlets may be arranged in an array.

The droplet generator may comprise a piezoelectric actuator which is configured to control the ejection of droplets from the at least one outlet.

The aeroponic propagator may comprise a root chamber and a foliage chamber. The foliage chamber may be separated from the root chamber. The fogging system may be configured to supply fog to the root chamber and/or the foliage chamber.

The droplet generator may be configured to eject droplets having a diameter of less than or equal to approximately 40 µm, or preferably less than or equal to 30 µm, or more preferably less than or equal to approximately 20 µm.

The droplet generator may be configured to eject approximately at least 1000 droplets per second from each of the outlets.

The aeroponic propagator may further comprise a reservoir of liquid for use by the droplet generator to generate droplets. A composition of the liquid may be selected to comprise at least one of RNA, a nitrogen fixing bacteria, and/or a hormone and/or chemical to promote detachment of produce from cultivated plants.

According to another aspect a method of supplying fog within an aeroponic propagator may be provided, the method comprising generating droplets to form the fog using a droplet generator comprising at least one outlet, wherein the generation step comprises generating one droplet of liquid at a time from each at least one outlet.

The fogging system may be configured to electrically charge the fog.

The aeroponic propagator may comprise a root chamber and a foliage chamber. The root chamber may be separated from the foliage chamber. The foliage chamber may be separated from the root chamber as will be described in further detail below. The fogging system may be configured to supply fog to the root chamber and/or the foliage chamber.

In use, the fogging system may be configured to electrically charge fog supplied to root chamber.

The fog supplied to the root chamber may be negatively charged.

In use, the fogging system may be configured to electrically charge fog supplied to the foliage chamber.

The fog supplied to the foliage chamber may be positively charged.

The aeroponic propagator may comprise at least one mesh and may be configured to pass the fog through the at least one mesh to alter the electrical charge of the fog.

The aeroponic propagator may comprise multiple meshes which are arranged such that fog being charged passes successively through each of the meshes.

The aeroponic propagator may comprise an elongated structure with a first end and a second end and the fogging system may comprise a blowing device configured to move the fog from the first end towards the second end. In this arrangement, multiple meshes may be arranged along the elongated structure such that fog is charged by each of the meshes as it is moved along the length of the elongated structure.

The fogging system may further comprise at least one blowing device configured to move the fog.

The aeroponic propagator may further comprise a reservoir of liquid for use by the droplet generator to generate droplets. A composition of the liquid may be changed and may be selected to comprise at least one of RNA, a nitrogen fixing bacteria, and/or a hormone and/or chemical to promote detachment of produce from cultivated plants.

The method may comprise electrically charging the fog.

The aeroponic propagator may comprise a reservoir of liquid for use by the droplet generator to generate droplets, wherein the liquid comprises RNA.

The aeroponic propagator may further comprise a root chamber, in which the roots of plants are disposed in use. The fog may be supplied to the root chamber.

The liquid may be lipophilic preferably with a range with a partition coefficient logP_{oktanol/water} between approximately -5 and 5 and more preferably between approximately 0 and 3.

The method may comprise providing a liquid and generating a fog within the aeroponic propagator using the liquid, wherein the liquid comprises RNA

The present invention will be described with reference to exemplary embodiments and the accompanying Figures in which:
Fig. 1 is a schematic perspective view of an aeroponic propagator which may be used with an embodiment of the present invention;
Fig. 2 is a schematic side view of the end of an aeroponic propagator which may be used with an embodiment of the present invention;
Fig. 3 is a schematic view of the end of another aeroponic propagator which may be used with an embodiment of the present invention;
Fig. 4 is a schematic end view of an aeroponic propagator according to the present invention;
Fig. 5 is a schematic view of a part of the droplet generator which may form part of a fogging system according to the present invention;
Fig. 6A is a schematic cross-sectional view of an aeroponic propagator according to the present invention;
Fig. 6B is a close up view of part of an electrically charging device depicted in Fig 6A;
Fig. 7 is a schematic end view of an aeroponic propagator according to the present invention.

In the Figures, like parts are identified by like reference numbers. The features shown in the figures are not necessarily to scale and the size or arrangements depicted are not limiting. It will be understood that the figures include optional features which are not essential to the invention. Furthermore, not all of the features of the aeroponic propagator are depicted in each figure and the figures may only show a few of the components relevant for a describing a particular feature.

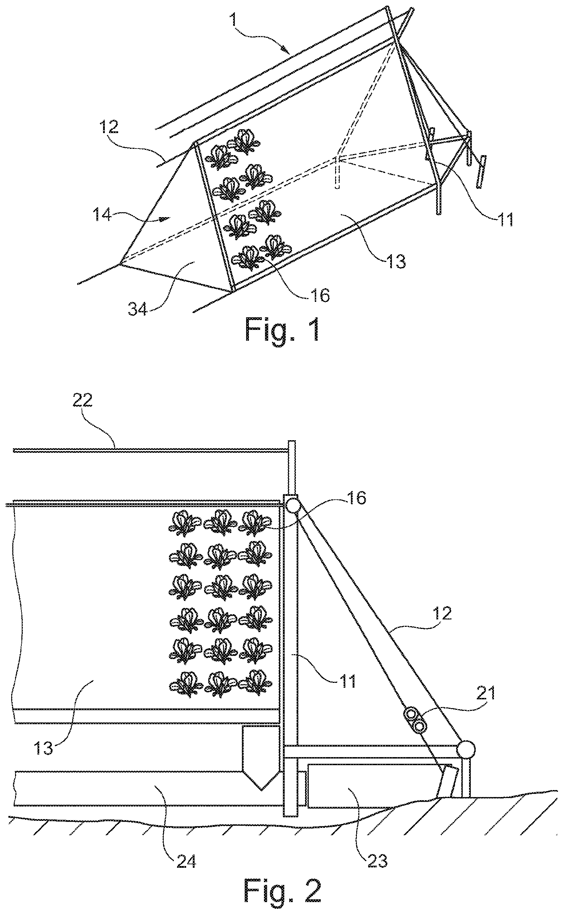

Figures 1-3 show various views of an aeroponic system, which is an example of an aeroponic propagator 1. The aeroponic propagator 1 is used for the cultivation of plants when in use. It is noted that the term plants is used as a general term to cover different species of plant and at least part of a plant, for example the roots and/or the foliage. Seeds may be used instead of plants and any of the embodiments described below may be used with seeds, thus the seeds may be germinated to form plants which are cultivated.

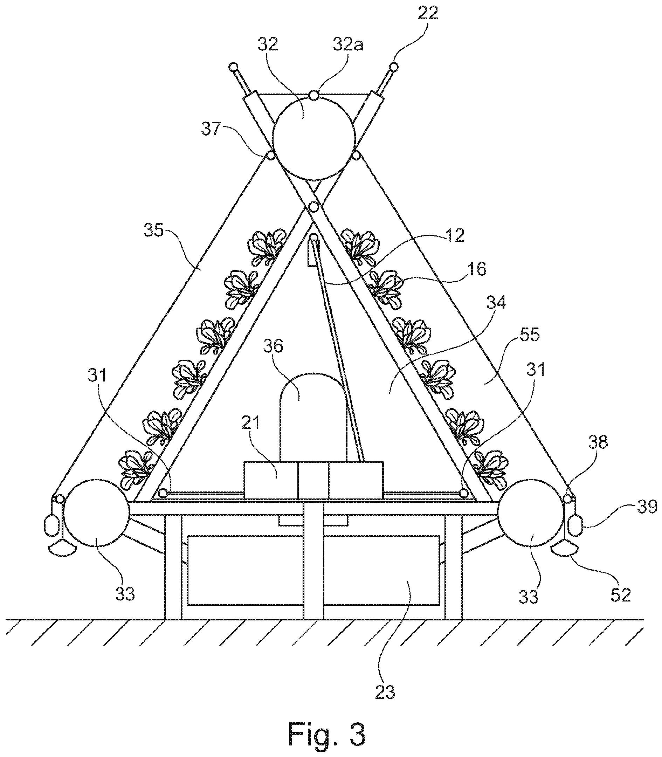

The system may be the same as described in WO 2012/156710 A1. The system may include the same features as in any of the embodiments of WO 2012/156710 A1 and may have any of the variations disclosed therein. For example, the aeroponic propagator 1 may be as depicted in Figures 1 to 3 which are described in detail in WO 2012/156710 A1. As described, the aeroponic propagator 1 may comprise a frame optionally comprising end frames 11 The frame may be covered by at least one sheet 13. The sheet 13 may define a space used as a root chamber 14. Plants 16 may be grown on the sheet 13 wherein the roots of the plant 16 may be located below the sheet 13 in the root chamber 14 and the foliage of the plant 16 may be located above the sheets 13. The aeroponic propagator 1 may comprise an arrangement of lines, such as line 12 which is used to hold the frame in position. The line 12 may further comprise at least one winch, such as winch 21 to keep the line 12 taught to provide support to the structure. Further lines may be provided depending on the structure of the aeroponic propagator 1, for example line 37 in Figure 3. The aeroponic propagator 1 may additionally comprise a closer panel 34 as depicted in Figure 1 to close the root chamber 14.

As depicted in figure 2, the aeroponic propagator 1 may comprise a fogging system 23 configured to generate a fog, and further may comprise a return tube 24 to extract fluid from an area of the aeroponic propagator, for example the root chamber 14. As also depicted in figure 2, the aeroponic propagator 1 may comprise power cables 22 to provide power to at least one component of the aeroponic propagator 1. For example, the power cables 22 may provide electricity to the fogging system 23.

Figure 3 depicts a further variation described in greater detail in WO 2012/156710. The aeroponic propagator 1 in figure 3 may be similar to the aeroponic propagator shown in figure 2, except the aeroponic propagator 1 in figure 3 also comprises a foliage chamber 55 in which the foliage of the plants 16 may be located as the plants 16 are cultivated. The aeroponic propagator 1 may comprises an outer sheet 35 which substantially defines the outer portion of the foliage chamber 55. The outer sheet 55 may be an outer sheet of the aeroponic propagator 1. The aeroponic propagator 1 may comprise a vent flap 36 which can be opened to allow fluid to pass into or out of the root chamber 14. Similarly a further vent flap (not shown) may be provided to allow fluid to pass into or out of the foliage chamber 55. Vent seals 32 and 33 may be provided, which may for example be inflatable vent seals, to control the passage of fluid into and out of the aeroponic propagator 1, or the root chamber 14 and/or the foliage chamber 55. The vent seals can attach to the propagator 1 via any suitable connecting mechanism, for example vent seal 32 may be connected via connecting mechanism 32a in Figure 3. Further lines, 37 and 38 may be provided as in Figure 3.

The aeroponic structure 1 may be supported in various means, for example, through the use of supporting members 31. The sheet 13 may be secured to the frame and/or lines e.g. line 38, via any appropriate method, such as strong acting clips similar to large plastics clothes pegs or by using the deadweight of water, such as in water-filled tubes 39 in Fig. 3 for example. Additionally, the aeroponic propagator may comprise gutters 52 as a means for collecting water, from condensation on the inside of the sheet 13 as well as rain water and dew. The water may be collected and recycled.

It is to be understood that these figures depict example structures of the aeroponic propagator 1. The aeroponic propagator 1 may have a substantially different shape, for example, it may be a cuboid, e.g. a rectangle, a cylinder, a sphere, a spherical cone or any other suitable shape. The foliage chamber 55 and the root chamber 14 may form part of the aeroponic propagator in various different ways not limited to the examples shown in the drawings. For example, the root chamber may be a bottom portion of the aeroponic propagator, irrespective of shape, and may take up any appropriate portion of the overall aeroponic propagator 1 depending on the size of the aeroponic propagator 1. The root chamber 14 and/or the foliage chamber 55 may comprise structures and or supports for the roots and/or foliage respectively as required.

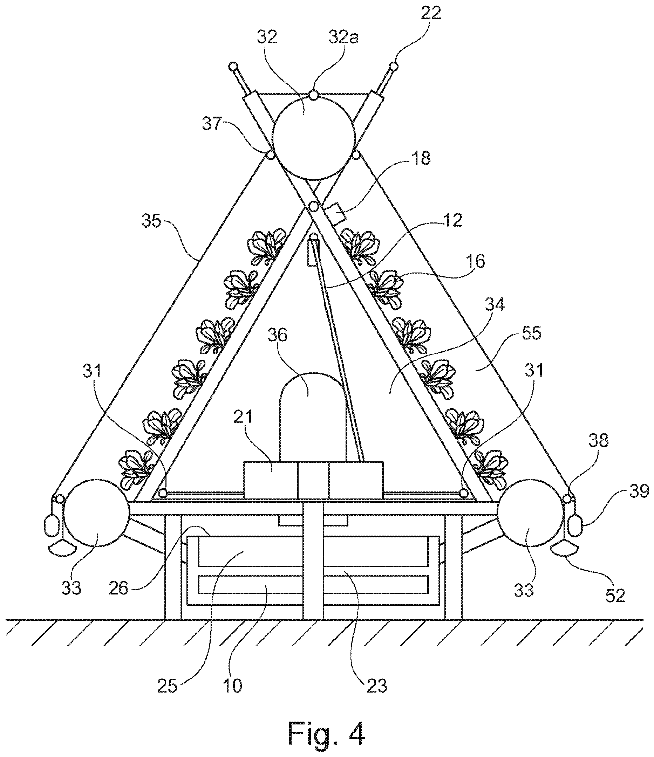

According to the invention, an aeroponic propagator 1 is provided, the aeroponic propagator 1 comprising a fogging system 23 depicted in figure 4. This may be the same as the fogging system 23 depicted in Figures 2 and 3. The fogging system 23 is for supplying a fog within the aeroponic propagator 1, the fogging system 23 comprising a droplet generator 25. The droplet generator 25 comprises at least one outlet 26. The droplet generator 25 is configured such that in use each at least one outlet 26 ejects one droplet of liquid at a time. This does not mean that every outlet 26 must eject a droplet at the same time, but means that every outlet of the droplet generator is configured to output only one droplet at a time. In this way, the droplet generator 25 may be any device capable of ejecting individual droplets. Thus, the droplet generator 25 outputs separate droplets of liquid, for example in a stream of separated droplets, from each outlet 26. Known droplet generators may include known print heads used in commercial inkjet printers, e.g. using Xaar ^{®} printheads. Thus, commercial print heads could be used as a droplet generator 25 in the aeroponic propagator 1 to generate the fog.

When the droplets are generated individually by outlets 26 ejecting one droplet at a time, there are specific advantages associated with the fog which is generated. Such a fogging system 23 can generate a fog in the aeroponic propagator 1. The fog is understood to be different to the mist/aerosol provided by prior known systems. Mist/aerosols generated in the prior art generally comprise larger droplets. Advantageously, an aeroponic propagator 1 as in the present invention can more accurately control sizes of droplets forming the fog by using such a droplet generator. For example, the fogging system 23 may be used to provide very small water droplets which in bulk form a dense fog. One of the main advantages of such a fog is that in high humidity environments it can stay in the air for long periods of time compared to mists/aerosols generated in previously known aeroponic systems. As such a fog will stay in the air for longer periods of time it can travel longer distances than mists/aerosols meaning it may be used to supply water and nutrients to a larger number of plants 16.

In known hydroponic systems, the nutrient concentration in the liquid is progressively reduced by plant roots as it travels along irrigation channels. However, in an aeroponic propagator 1 as described above, the level of nutrient and pH in individual fog droplets may remain constant irrespective of the distance travelled because until a droplet is absorbed, it will substantially maintain the level of nutrient and pH. This means that plants may be provided with the same nutrients and pH level irrespective of the location relative to the supply of nutrients and pH. Furthermore, this provides additional control of nutrients and pH because these levels in the droplets can be controlled/changed and this may be affected throughout the aeroponic propagator quickly allowing fast response to desired changes.

Using a droplet generator 25 as in the first embodiment allows control of the droplet size and thus, the droplet size can be adapted as desired. For example, the size of the droplets may be made relatively small (i.e. with a small diameter) compared to mist/aerosol droplets. The relatively small droplets may be used to create what is generally known as "dried fog". It is known that using mist droplets (which are of a larger size) can saturate the minute closely spaced root hairs which may prevent air and a fresh supply of nutrients and may prevent droplets penetrating uniformly among the plant roots. The resulting lack of oxygen and nitrogen may inhibit plant growth and can in extreme circumstances cause the plants to die. In the prior art, mists/aerosols are normally generated using a nozzle and/or spray. The nozzle and/or spray generate multiple droplets at a time which does not provide the same control over the size of each of the droplets as is provided by the droplet generator 25 of the present invention. Generating such a dry fog as in the first embodiment can be advantageous because the dry fog does not saturate plant roots and therefore, the roots do not become wet or drip water. Thus, the aeroponic propagator 1 having the droplet generator 25 reduces or prevents these problems compared to using know propagator systems which generate mists/aerosols.

The fogging system 23 of the first embodiment may have many outlets 26. For example, instead of at least one outlet 26, there may be at least approximately 100 outlets 26, or at least approximately 300 outlets 26 or at least approximately 1000 outlets 26 or even more. The number of outlets 26 may be specifically chosen depending on desired characteristics of the fog and/or the size of the aeroponic propagator 1. It may be beneficial to have a fogging system 23 configured to make multiple, even a large number of droplets per second from many outlets. This would improve the speed of generation of the droplets.

The fogging system 23 is configured to provide the fog within the aeroponic propagator 1 such that the fog is within at least a part of the aeroponic propagator 1. In this way, the fog can be provided to the plants 16 to be cultivated, whether or not it is the root, the plant, or both, or even the seed before the plant is germinated. As will be described in further detail below, different characteristics of the fog may be controlled by using the droplet generator 26 to provide further benefits.

As depicted in Figure 4, the aeroponic propagator 1 may comprise a root chamber 14 and optionally a foliage chamber 55. The root chamber 14 may be separated from the foliage chamber 55, for example, by a sheet, such as sheet 13 depicted in Figures 1, 2 or 3. The root chamber 14 and the foliage chamber 55 may be separated in that a physical barrier is provided between the two chambers as depicted in Figures 3 and 4. However, the root chamber 14 and the foliage chamber 55 may be separated in that a physical barrier is provided to reduce interaction between fluids in each of the chambers. In other words, the separation between the root chamber 14 and the foliage chamber 55 may not completely isolate the root chamber 14 from the foliage chamber 55 and vice versa. The separation may be provided by a sheet, similar to sheet 13 which still allows some interaction of gases between the two chambers. It would be understood that the purpose of the separation is to control the fluid provided to different parts of the plants, i.e. the roots or the foliage and that it is not necessary to completely isolate the root chamber 14 from the foliage chamber 55 in order to control the environment in each of the root chamber 14 and the foliage chamber 55 to an adequate degree.

The fogging system 23 may be configured to provide fog to the root chamber 14, the foliage chamber 55 or both. Advantageously, this allows fog to be supplied to the root chamber 14 and the foliage chamber 55 at different times and may also allow different types of fog to be supplied to the different chambers. This beneficially provides further control, for example, the droplet size and/or liquid used to form the droplets may be different in each of the root chamber and the foliage chamber. This increases the level of control available which may improve the growth of plants 16 being cultivated within the aeroponic propagator 1.

The fogging system 23 may be controlled to adapt the fog depending on which chamber or chambers the fog is being provided to. For example, at least one sensor 18 may be provided in the foliage chamber 55 as depicted in Figure 4 and the fogging system 23 may be dynamically controlled to alter characteristics of the fog provided to the foliage chamber 55 depending on feedback from the at least one sensor. For example, the rate of droplets being ejected by the fogging system 23 may be altered, or the liquid used by the fogging system 23. Additionally, or alternatively a sensor such as sensor 18 may be provided in the root chamber 14. Thus, the fogging system 23 may be similarly dynamically controlled whether providing fog to the root chamber 14, the foliage chamber 55 or both. The at least one sensor 18 may be configured to measure a characteristic used for dynamic control, such as a characteristic of the environment in the respective chamber, e.g. the humidity in the respective chamber, the temperature, etc.. Additionally, or alternatively, the sensor 18 may be configured to measure a characteristic of at least one of the plants 16 rather than the environment in the respective chamber surrounding the plant.

The fogging system 23 may comprise multiple outlets 26, i.e. the fogging system 23 may comprise plural separate outlets 26, each of which are configured to provide droplets of liquid one droplet at a time. This can be beneficial in providing a large number of droplets because multiple outlets 26 means that the number of droplets provided at any one time can be increased. Thus, the number of droplets, and therefore, the size of the fog may be controlled depending on how each of the plural outlets 26 are controlled.

The plural outlets 26 may be provided in an array. In other words the outlets 26 may be provided in some sort of matrix formation. Providing the droplets in an array allows the formation of the fog to be controlled. This also allows the fogging system 23 to have the capacity to create a dense fog. For example, the multiple outlets may be arranged in a desired pattern, or with a specific spacing, to aid formation of a fog having a preferred density. The density may affect the movement of the fog and the interaction with the plants, such that controlling these characteristics is beneficial. The array may or may not be linearly organised and may or may not be arranged in a regular pattern formation. The array may be arranged to provide the fog in such a way that air and/or fog is moved in spiralling vortices which may focus fog movement. This may be similar to the way vortex cannons function. This may be useful in root chamber 14 and/or the foliage chamber 55 as a way to increase the distance the fog travels and to improve fog penetration in closely spaced root foliage areas.

The droplet generator 25 (comprising any suitable number of outlets) may be configured to eject approximately 1000 droplets per second from each at least one outlet. It may be possible and preferable to eject many more than this, for example thousands, tens or hundreds of thousands, and millions or even tens or hundreds of millions. The number of droplets ejected by each of the outlets 26 per second may be controlled or varied as desired. This advantageously allows the environment surrounding the roots and/or foliage to be varied as desired.

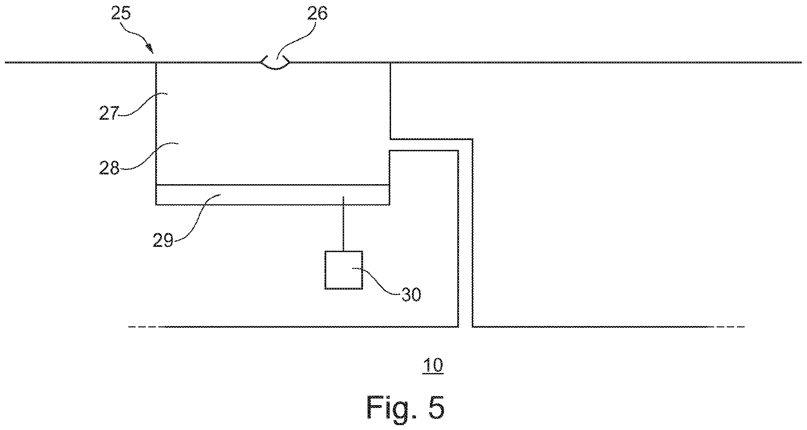

The droplet generator 25 may comprise a piezoelectric actuator 29 as depicted in Figure 5. Figure 5 depicts a more detailed version of the droplet generator depicted in Figure 4. The use of piezoelectric actuators are known in other fields, such as in inkjet printers, and have the advantages that they are commercially available, can be used to generate droplets at a high frequency, have a high degree of accuracy (i.e. control) and efficiency, and are reliable. Additionally, the accuracy and line-up of holes is extremely important for printing, but the tolerances are not as high in the aeroponic propagator 1 such that recycled or rejected piezoelectric actuators (i.e. piezoelectric or other appropriate printheads) may be used which provide enough accuracy in the aeroponic propagator 1. The accurate control of droplet size is not possible using prior known aeroponic systems, using for example sprays/nozzles to generate mists/aerosols, because multiple, possibly large numbers of, droplets are generated at the same time. The individual generation of droplets using a piezoelectric actuator 29 allows accurate control the droplet size which is particularly beneficial compared to known aeroponic systems. The droplet generator 26 may comprise commercially available print heads (for example to use instead of the system shown in Figure 5). Commercially available print heads are more reliable and at less cost compared to conventional spray nozzles used in aeroponic systems/for agricultural purposed. The reduced cost may be both in terms of capital cost of each print head unit (versus conventional nozzles) and operational cost as the print heads do not require any significant pressure drop (which is required for conventional nozzles) to generate the fog.

The piezoelectric actuator 29 may be configured to control the ejection of droplets from the at least one outlet 26. The piezoelectric actuator 29 may comprise a component made from piezoelectric material. A control system 30 may further be provided, wherein the control system may be configured to control the application of an electrical current to the piezoelectric actuator 29 such that the piezoelectric material changes shape. The application of an electrical current to the piezoelectric material may cause the piezoelectric material to change shape, most likely to expand rapidly which changes the pressure inside the cavity 27 and which may cause an individual droplet of liquid 28 to be ejected from the at least one outlet 26.

The droplet generator 25 may comprise at least one cavity 27 which retains liquid 28 as depicted in Figure 5. The cavity 27 may have a single outlet 26 or multiple outlets. The outlet 26 allows the liquid 28 to be ejected from the cavity 27. The piezoelectric actuator 29 may be formed such that a change of shape of the piezoelectric material alters the space confined by the cavity 27. For example, the piezoelectric material may form at least a part of a wall of the cavity 27, or may be situated in the cavity 27 (in the liquid 28, when in use), or may be formed in a wall of the cavity 27 wherein the wall is capable of deforming in response to a change of shape of the piezoelectric material. The aeroponic propagator 1 may comprise a reservoir 10 which can be used to retain the liquid 28. The location of the reservoir 10 may be selected depending on the structure of the aeroponic propagator 1 and how the reservoir 10 is used to supply liquid 28 to the cavity 27. In an embodiment, the reservoir 10 may be part of the droplet generator 25 or the fogging system 23 more generally. However, the reservoir 10 could be external to the fogging system 23 and may supply liquid 28 to the fogging system 23 in order to generate the fog, i.e. the reservoir 10 may be in fluid communication with the fogging system 23. The reservoir 10 may therefore, be used to provide liquid 28 to the at least one cavity 27. In an alternative example, the reservoir 10 may be understood to mean the cavity 27 within the droplet generator 25 as this cavity 27 is capable of holding and storing the liquid 28.

Alternative methods of controlling the droplet generator may also be provided. For example, thermal excitation may be used to move droplets of liquid 28 from the a cavity 27 (not depicted). This may comprise the use of at least one heater in a chamber in the droplet generator 25. A control system similar to the control system 30 used with the piezoelectric actuator 29 may be provided. The control system may be used to control a current being passed through the heater which causes rapid vaporization of liquid in the chamber which forms a bubble. The bubble leads to an increase in pressure in a corresponding cavity of which causes a droplet of liquid to be ejected from the droplet generator 25.

It is understood that different types of ejection unit might be used i.e. the piezoelectric actuator 29 may be replaced, for example, using a thermal excitement unit as described above, or with other types of actuators or units. For example, the piezoelectric actuator 29 may be replaced with a system using thermal jet printer technology, an actuator comprising magneto-restrictive material, an actuator comprising an electromagnet (e.g. an electromagnetic coil), a unit using resistive heating, and/or a unit using deformation and/or a response to voltage (e.g. any system which makes use of a voltage) etc.. Many known single or multiple commercially available units may be used which provide an individual drop of liquid at a time. For example, a droplet generator as described in WO 2010/055344 A1 could be adapted for use in the aeroponic propagator 1.

It may be advantageous to eject droplets having specific sizes. The droplet generator 25 may be configured to eject droplets having a diameter of less than or equal to approximately 40 µm, or preferably less than or equal to approximately 30 µm, or preferably less than or equal to approximately 25 µm, or more preferably less than or equal to approximately 20 µm. It is preferable to use droplets below these values to prevent the droplets becoming too heavy and "dropping" out of the fog. The droplets may be as small as approximately 0.5 µm, or even approximately 0.1 µm. Thus, the droplets may have diameters in the range of approximately 0.1 µm to approximately 40 µm, or preferably from approximately 0.5 µm to approximately 30 µm.

The minimum size droplets that can be used may depend on the reliability and economic viability of the technology used to generate the droplets. The reduced diameter can be beneficial in more efficiently providing the desired amount of liquid to the plants. For example, if the droplets are made small enough (e.g. with a diameter of less than or equal to approximately 30 µm, or more preferably 20 µm, or even as small as 0.1 µm or 0.5 µm), they may be able to pass through stomata guard cells directly into the plants. Stomata are the cells on the undersides of leaves that plants "breathe" through, i.e. they take in carbon dioxide and release oxygen through the stomata. This is potentially a highly effective way to feed plants and introduce liquid in the fog into plants. Thus, the aeroponic propagator 1 with droplets having the above sizes allows plants to be foliar fed in rapidly moving currents of dense nutrient-containing liquid fog. The fog may have droplets which are small enough to penetrate the open guard cells of stomata. The fog can create high humidities in the aeroponic propagator 1 which allow stomata to open for gas exchange and nutrient contained in the fog droplets to easily penetrate the leaves and produce a uniquely strong foliar feed action.

The invention also concerns a method of supplying fog within an aeroponic propagator 1, the method comprising generating droplets to form the fog using a droplet generator 25 comprising at least one outlet 26, wherein the generation step comprises generating one droplet of liquid at a time from each at least one outlet 26. The method of generating the fog may use any of the above described systems or apparatus. Thus it is understood that the method may generate a fog as described above (for example, having the preferred droplet sizes, etc.)

The fogging system 23 may be configured to electrically charge the fog. Providing a charged fog within the aeroponic propagator may help stimulate growth and control harvest timings of plants being cultivated by the aeroponic propagator. Although the idea of stimulating and/or controlling the growth of plants 16 using electricity is known, the problem with electrical growth stimulation in the past has always been that they depended on stimulating plants 16 which were grown in soil. In soil, conditions can often vary greatly between one part of a field to another, for example, due to chemical imbalances and/or wood content. The variations affected the electrical conductivity of soils in ways that were unpredictable and therefore, impossible to allow for. More generally, there have been barriers to the use of electrical plant growth for various reasons including the limitation of growing plants in soil, a lack of control over growth conditions (e.g.in leaf canopies and root areas), and high capital and operating costs. Using an aeroponic propagator 1 as in the present invention has the advantage that the charge applied to the plants 16 throughout the propagator may be more uniform than in previously known systems which apply a charge to the plants.

Advantageously, the aeroponic propagator described above uses fog to deliver water and plant nutrients to the plants to be controlled. By electrically charging the fog, the fog droplets generally repel one another, so tendency for droplets to aggregate and become mist droplets is reduced or prevented. As previously described, smaller droplets may be capable of travelling further, for example, when compared to larger mist droplets. Thus charged fogs may travel relatively long distances in a higher humidity. Additionally, charging the fog greatly enhances the potential fire distinguishing performance of the fog which is very useful as a safety mechanism in certain emergency scenarios, such as if lightening strikes the aeroponic propagator.

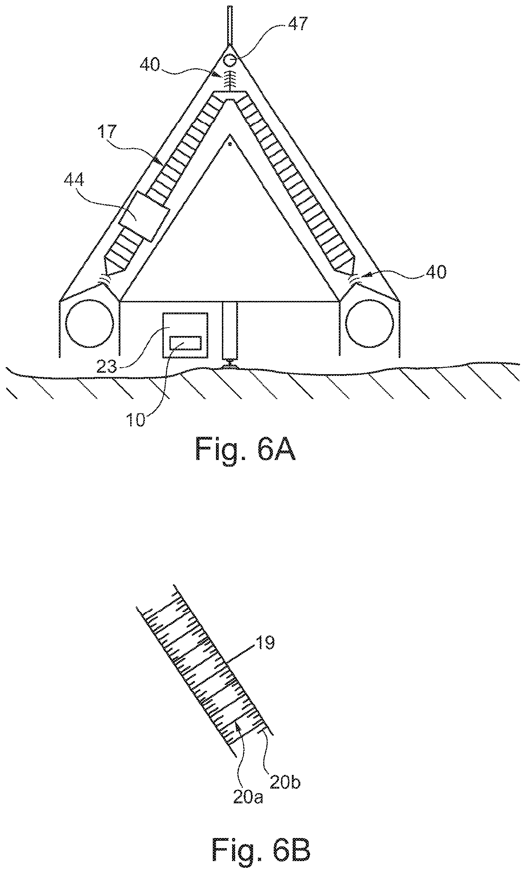

The charge may be supplied in a number of ways. The fogging system 23 may comprise at least one electrically charged device configured to supply a charge to the fog supplied to or within the aeroponic propagator 1. The aeroponic propagator 1 may comprise at least one transformer 47, preferably at least one small low-cost high voltage transformer. The transformer 47 may be connected to electrically conductive components (e.g. rods or meshes as described below). The electrically charged device may be any shape. An example of an aeroponic propagator 1 configured to electrically charge the fog is depicted in figure 6A.

In this example, the electrically charged device may be a conductive structure 17 as depicted in figure 6A. The conductive structure 17 may include at least one metal rod 19 (which may instead be a metal rope, or any conductive rod or rope). Additionally, the electrically charged device may comprise links 20a between two metal rods, as depicted in Figures 6A and 6B. The links 20a may be metal or carbon fibre strands connected to at least two metal rods 19. Additionally or alternatively, fibres such as fibre 20b may be provided along the length of at least one metal rod 19. These fibres 20b may be shorter than the links, but may also be formed by metal or carbon fibre strands. Each of the fibres 20b may only be connected to one metal rod 19. These links 20a and fibres 20b are used to increase the available surface area of the conductive structure to more efficiently electrically charge the droplets of fog as they pass through the conductive structure 17. Instead of a conductive structure 17, the electrically charged device may be a mesh (not shown). The fogging system may supply fog to the area containing the electrically charged device. In an example, at least one insulator 40 may be provided with the electrically charged device in order to maintain the charge in the desired area and to keep the aeroponic propagator 1 safe for use.

In this embodiment, the aeroponic propagator 1 may comprise a root chamber 14 and/or a foliage chamber 55 substantially as described above (i.e. the root chamber 14 and the foliage chamber 55 may be separated as described above). Advantageously, this allows fog to be supplied to the root chamber and the foliage chamber at different times and may also allow different types of fog to be charged differently depending on the chamber it is being supplied to. The electrically charged device may be located in either chamber. For example, as depicted in figure 6A, the conductive structure 17 may be located in the foliage chamber 55.

In this embodiment, the aeroponic propagator 1 may be configured to pass the fog through the electrically charged device to alter the electrical charge of the fog. Thus, the aeroponic propagator 1 may pass the fog through the conductive structure 17 depicted in figure 6A, (or through a mesh used alternatively or additionally to the conductive structure 17). Multiple electrically charged devices may be used. For example, the electrically charged devices may be provided at various different locations throughout the aeroponic propagator 1. This is beneficial because it means that the electrically charged devices can supply an electrical charge to fog located in different areas of the aeroponic propagator 1.

In an example, multiple electrically charged devices may be provided and the aeroponic propagator 1 may be configured to pass the fog through the electrically charged devices successively. This may mean that the aeroponic propagator 1 is arranged to pass the fog though all of the electrically charge devices, and/or that the aeroponic propagator is configured to pass the fog through multiple electrically charged devices one after the other. This allows the charge to be changed, for example increased, as the fog passes through each of the electrically charged devices.

In any of these examples or embodiments, the fogging system 23 may comprise a blowing device 44. The blowing device 44 may be a simple motorised fan, or it may be a more complicated system. The blowing device 44 may be configured to move the fog through the aeroponic propagator 1 in a desired direction. The blowing device 44 may be controlled to have a variable effect, for example, the blowing device may have variable intensity and/or direction to allow more specific control over the fog. For example, the blowing device 44 may be a variable speed waterproof fan. The blowing device 44 may be used in conjunction with a sucking device (not depicted) which may also, for example, comprise a fan. The blowing device 44 and the sucking device could be arranged to work in conjunction with each other, for example, the blowing device 44 may be located on one side/end of the aeroponic propagator 1 and the sucking device may be located at the other end of the aeroponic propagator 1 to suck more fog and air through the aeroponic propagator thus improving movement of fog and/or air though the aeroponic propagator 1.

In a preferred arrangement, the aeroponic propagator 1 might have a shape which is be substantially elongated. For example, the aeroponic propagator 1 may be as described in some of the examples above and as depicted in figure 1, i.e. the aeroponic propagator 1 may be a cylinder or a cuboid, etc.. In this embodiment, the elongated structure may have a first end and a second end. The fogging system 23 may be configured to supply the fog to the aeroponic propagator substantially towards one end. Although ideally, this may mean that the fogging system 23 supplies fog near the first or second end, it may more broadly mean that the fog is supplied in approximately a first half of the aeroponic propagator 1. The fogging system 23 may comprise a blowing device 44 configured to move the fog towards the other end. This may mean that the blowing device 44 is configured to move the fog in a direction towards one end and away from the other.

In this arrangement, multiple electrically charged devices may be arranged along the elongated structure, i.e. along the length of the elongated structure, such that fog is charged by each of the electrically charged devices as it is moved along the length of the elongated structure. As the fog is moved by the blowing device 44 substantially along the length of the aeroponic propagator, the fog may pass through spaced out electrically charged devices. Thus, the electrical charge of the fog may be controlled as it passes through each of the electrically charged devices. For example, the electrically charged devices may be used to "boost" the electrical charge of the fog as it passes through each of the electrically charged devices.

When the fog is charged at a single location, the charge may decrease as the fog moves through the propagator. However, when multiple electrically charged devices are provided as in some of the examples above, this may be advantageous because the charge of the fog can be effectively controlled (e.g. increased) as the fog passes through each electrically charged device. Thus, the fog may be provided with a more consistent charge such that the same charge reaches foliage and/or roots no matter where they are located in the aeroponic propagator 1. In other words, using multiple electrically charged devices, ideally arranged in the preferred arrangement, can provide more uniformly charged fog to the plants 16 throughout the aeroponic propagator 1.

In this embodiment, fog may be supplied such that the charge of the roots and the foliage are different form each other. For example, a fog may be supplied to the root chamber 14 which is negatively charged. Additionally or alternatively, no fog, or a positively charged fog may be supplied to the foliage chamber 55. It may be particularly beneficial to use the fog to generate a negative charge on the roots and a positive charge on the foliage because this may lead to more efficient passing of the droplets into the stomata as it takes account of the natural potential across the plant. Alternatively, a fog may be supplied to the foliage chamber 55 which is negatively charged. Additionally or alternatively, no fog, or a positively charged fog may be supplied to the root chamber 14. No matter the variation, the charge of the roots and the foliage may preferably be controlled such that the charge of the roots and the foliage are different from each other (although this is not required). This may improve the efficiency of the droplets passing into stomata on the leaves.

As described in the above embodiments, the fogging system 23 may comprise a reservoir to hold the liquid used by the fogging system 23 to generate the fog. This reservoir may be the same reservoir 30 as described above. The reservoir may be provided in a further embodiment or with any of the embodiments described above. For example, the reservoir may be within the fogging system 23 depicted in figures 2 and 3. Alternatively the reservoir may be attached and in fluid communication with the fogging system 23 to provide liquid to the fogging system 23 to generate the fog.

The method of supplying fog within an aeroponic propagator, comprising generating a fog within the aeroponic propagator, may further comprise electrically charging the fog. The method of generating the fog may use any of the above described systems or apparatus. Thus it is understood that the method may generate a fog as described above (for example, using a piezoelectric actuator to electrically charge the fog, etc.)

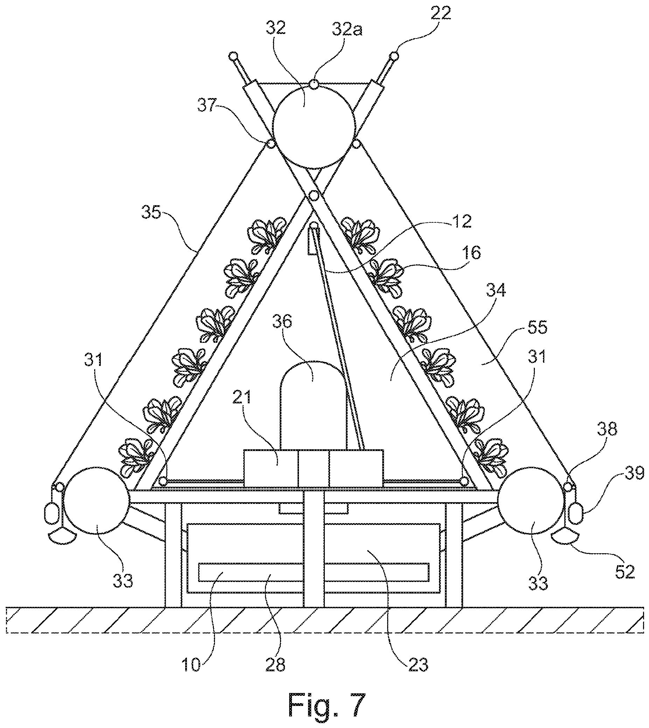

The aeroponic propagator 1 may comprise a reservoir 10 as depicted in Figure 7. The reservoir 10 may be the same as the reservoir described. The reservoir depicted in Figure 7 is part of the fogging system 23, however, the reservoir 10 may be external to the fogging system 23 and may be in fluid communication with the fogging system. The reservoir 10 of liquid 28 is for use by the droplet generator to generate droplets. The reservoir 10 is simply a part of an apparatus which may be used to hold the liquid 28. The liquid 28 comprises Ribonucleic acid (RNA).

Conventionally applied spray droplets (e.g. applied by spray devices) fall from above onto leaf surfaces, but rarely reach their undersides where stomata are located. For applying herbicides, and pesticides this need not always be a problem, but in the case of RNA molecules in sprays it is because they must enter stomata in order to have the desired effect. Thus using RNA in an aeroponic propagator as in the present invention is more effective at delivering RNA and the RNA can be provided in much lower quantities than needed by conventional agricultural spray methods. Using RNA in an aeroponic propagator may be more effective because spray drift is not a problem, high humidity conditions mean stomata are most likely to be accessible, the air speed can be controlled and also due to Brownian motion of air and water molecules.

Genetically modified organisms (GMO) are under suspicion by large parts of the population, as the DNA of an organism is changed, and so is its offspring, thus creating new "species". An alternative to alter behaviour without changing genetic properties is to "dope" an organism with RNA. RNA is the messenger code that organisms use to switch or enhance cell activities. Generally, injecting RNA into plants is not economically viable because it is expensive and because the cell boundaries of leaves and stems are highly impermeable to RNA. Spraying RNA on leaves does allow some RNA to enter through the stomata, but most of the RNA is wasted. Soaking roots in RNA solution is very effective for at least certain types of RNA, but there is also not an economically viable option for doing this for field grown crops.

In the present embodiment, the RNA is advantageously delivered in an effective manner by the fog created by the fogging system in the aeroponic propagator. Thus the above aeroponic propagator advantageously provides RNA in a manner which can effectively and economically enter the root system of plants.

RNA interference is a way to temporarily turn off the activity of any gene. RNA interference is temporary and generally lasts for only a few days to maximum weeks, and is not permanent like GMO. RNA can regulate draught resistance, pest resistance, pesticide resistance, vegetative or generative growth stimulation, cell diversification and stimulation, and generally any kind of cell metabolism and growth.

Apart from ethical issues, a new GM crop can cost more than € 100 million and can take 13 years before it can be taken to market. Prices of RNA have come down from several thousands to several tens of Euro's per gram, which brings them in the range of economic application.

According to Monsanto patents the technique of spraying RNA also involve spraying a silicone surfactant to let RNA molecules slip into stomata, i.e. air-exchange holes, in the plant's surface. From the leaves, the RNA was transported through the entire plant. A similar, but essentially different, method is presented here by providing the RNA in a fog provided by a fogging system of an aeroponic propagator 1.

The aeroponic propagator 1 may comprise a root chamber 14 and/or a foliage chamber 55 substantially as described above (i.e. the root chamber 14 and the foliage chamber 55 may be separated as described above). The fogging system 23 may be configured to supply fog to the root chamber and/or the foliage chamber with the advantages described above.

The fog may be supplied to the root chamber 14. Supplying fog to the root chamber 14 means that the fog can effectively deliver RNA to a root system in the aeroponic propagator 1. Nutrients are taken up by the roots, and would be a way to use nature's normal transportation processes to deliver RNA. Known methods using RNA spraying have shown to be rather inefficient (and thus cumbersome and costly), and known methods of root delivery in soil-based production systems is impractical. However, supplying RNA using the fogging system 23 of the aeroponic propagator can be used without adaptation.

It is known that foliar feeding is possible but with the fog generator described in previous embodiment with a droplet size less than or equal to approximately 20 µm, and preferably less than 2 µm, and the protected environment (namely 100% or close to 100% humidity) of the growth chamber 55, foliar feeding is expected to be significantly more effective as will allow the nutrients and/or RNA to be absorbed directly into the plant's stomata as should be able to control the environment such that the stomata is open (e.g. stomata closes with low humidity).

The liquid may be lipophilic. In other words, the liquid may be "oil loving" i.e. hydrophobic. The liquid may preferably have a partition coefficient logP_{oktanol/water} between approximately -5 and 5 and more preferably between approximately 0 and 3. The liquid has this preferable range in the more lipophilic range of the spectrum because this can enable good transport through the lipid bi-layer of a plant cell boundary as well as through its cytosol.

Plants use minerals from the soil to build the complex molecules they need to survive and grow. As nutrient concentrations are usually higher inside the root than outside, plants normally use chemical energy to transport the nutrients inside. Covered by cortex outside cells, the "casparian layer" forms a corky barrier for which specialised carriers molecules are needed to cross. Adenosine triphosphate (ATP), the energy molecule of all cells, is used here, and the fact that roots cannot live without oxygen indicates the effort the plant has to do to absorb minerals. Once passed the casparian strip, nutrients move into the plant xylem tissue and are carried throughout other parts of the plant. To cross the casparian strip, nutrients have to cross through the cell plasma (symplectic transport vs apoplectic transport through the membranes). Normal cell walls consist of lignin, and phenolic and lipophilic biopolymers. In the Casparian strips these layers are so thick that they block submicroscopic capillaries in the primary cell wall and reduce apoplastic transport. Thus, this tight barrier regulates the apoplastic pathway, and solutes must move through the selectively permeable plasma membrane into the cytoplasm. As the membrane is made up of biopolymers like lignin (x-linked phenol polymer), and has a lipid nature, water and ions have to be let in by carrier molecules. Special carrier molecules exist for calcium, magnesium, copper, zinc and other minerals. This explains how plant roots can be selective in the nature of ions admitted into the plant roots.

Larger molecules, like organic molecules, can penetrate the lipophilic wall by diffusion. RNA may pass into the plant more efficiently if it is lipophilic or bonded to a lipophilic carrier (nano-droplets or lipodoids). Once passed the Casparian strip, the RNA can be transported through the cell plasma and to other cells through the plasmodermata. In this fog the RNA may be made lipophilic either by emulsification or as colloidal nanoparticle inside an "oily" nanoparticle, i.e. a lipodoid. The liquid being lipophilic means that the RNA (non-polar) molecules have to bond to the cell boundary to be transported inside the cell.

The fog may comprise very fine droplets. For example, the diameter of the droplets may be less than or equal to approximately 30 µm, or preferably less than or equal to approximately 25 µm, or more preferably less than or equal to approximately 20 µm. It is preferable to use droplets below these values to avoid the droplets becoming too heavy as described above. It is beneficial to have the droplets as small as this so as to defy gravity and stay afloat for long distances and time. To enhance attraction to the roots of the plants 16, the liquid may comprise surfactants, and the fog might be electrically charged (for example, as described above).

The method of supplying fog within an aeroponic propagator 1, the method comprising providing a liquid and generating a fog within the aeroponic propagator using the liquid, may be such that the liquid comprises RNA. The method may transport RNA in an aeroponic growth system in a fog to the root system to be absorbed there. The method may use the fogging system 23 and aeroponic propagator 1 described in any of the above embodiments and examples. The method may comprise containing liquid in a reservoir 10 to be used by a droplet generator to generate the fog.

The method may comprise generating a fog with an average particle size (i.e. droplet diameter) of less than or equal to approximately 25 µm or less, or preferably with an average particle size of less than or equal to approximately 5 µm. In an example the RNA may be mixed with water. The RNA may be mixed with water as solution, emulsion, colloidal or other means of dispersing RNA in water to be used as fog and is not necessarily homogenized. The fog may be allowed to travel passively e.g. by gravity or diffusion, and/or actively e.g. by a mechanical blower (such as the blowing device 44 described above). The fog may travel towards and on roots in the soilless root chamber 14 of the aeroponic propagator 1. The RNA may be engineered or selected to enter the cell root system. The RNA may be siRNA or miRNA, and may be enhanced with a lipid ("oily") tail with the sole purpose of the tail enhancement the entering of the root barriers.

In any of the above embodiments, the fog may be allowed to travel passively e.g. by gravity or diffusion, and/or actively e.g. by a mechanical blower (such as the blowing device 44 described above). Thus, any of the above embodiments and examples may comprise a blowing device, for example blowing device 44 described in relation to the second embodiment. The blowing device 44 may be used to control the speed of the fog and/or its direction and may be variable. Ideally, for example, the fog within the aeroponic propagator 1 will travel at a speed of approximately 1 m/s to 1.5 m/s, or preferably approximately 1.3 m/s. The fog being moved at this speed means that it is able to respond much more quickly to changing growth conditions than hydroponic systems can. The fog used in the foliage chamber 55 for example, is intended to disrupt the boundary layer of still or slow moving fogged air in contact with leaf surfaces in order to enhance leaf gas exchange processes. The fog used in the root chamber 14 for example, may be slower than in the leaf chambers for example, at a speed of 0 to 1.5 m/s to avoid chilling the roots and drying the surfaces of root hairs, which should always be kept either wet or moist - preferably the latter. It is noted that these speeds are preferable but the fog may be effectively used at lower or higher speeds also.

The liquid 28 used by the droplet generator 25 to generate droplets in any of the above embodiments may be selected as desired. As already described, the liquid 28 may comprise RNA. Further more, the liquid 28 may additionally or alternatively comprise nitrogen fixing bacteria and/or a chemical and/or hormone to promote detachment of produce from cultivated plants 16. The aeroponic propagator 1 may further comprise a reservoir 10 as described above to hold the liquid 28 for use by the droplet generator 25 to generate droplets.

Plant growth promoting bacteria (PGPB) have become increasingly important in the agricultural production of certain crops. However, the commercialisation and utilisation of PGPB has been currently limited due to the fact that there have not been consistent responses in different host cultivars and at different field sites. Thus, the controlled delivery of PGPB to root systems in soil is not possible under field conditions. The aeroponic propagator 1 of any of the above embodiments or examples can be used to generate a fog which can can be used to deliver nitrogen fixing bacteria (e.g. rhizobacteria) to plants which overcome that limitation. The use of plant growth-promoting bacteria in agriculture in general looks very promising, particularly when used in a fog generated by any of the aeroponic propagators 1 described above.

## Claims

1. An aeroponic propagator (1) for the cultivation of plants, the aeroponic propagator (1) comprising:
a fogging system (23) for supplying fog within the aeroponic propagator, wherein the fogging system (23) comprises a droplet generator (25), the droplet generator (25) comprising at least one outlet (26), and **characterised in that** the droplet generator (25) is configured such that in use each at least one outlet (26) ejects one droplet of liquid at a time.

2. The aeroponic propagator (1) of claim 1 wherein there are multiple outlets, and the outlets are arranged in an array.

3. The aeroponic propagator (1) of either of claims 1 or 2, wherein the droplet generator (25) comprises a piezoelectric actuator (29) which is configured to control the ejection of droplets from the at least one outlet (26).

4. The aeroponic propagator (1) of any one of the previous claims, wherein the droplet generator (25) is configured to eject:
i) droplets having a diameter of less than or equal to approximately 20 µm; and/or
ii) approximately at least 1000 droplets per second from each at least one outlet.

5. The aeroponic propagator (1) of any one of the previous claims, wherein the aeroponic propagator comprises a root chamber (14), and a foliage chamber (55) separated from the root chamber (14), and the fogging system (23) is configured to supply fog to the root chamber and/or the foliage chamber.

6. The aeroponic propagator (1) of any one of claims 1-4, wherein in use the fogging system (23) is configured to electrically charge the fog.

7. The aeroponic propagator (1) of claim 6, wherein the aeroponic propagator comprises a root chamber (14), and a foliage chamber (55) separated from the root chamber (14), and the fogging system (23) is configured to supply fog to the root chamber and/or the foliage chamber, optionally wherein in use the fogging system (23) is configured to electrically charge fog:
i) supplied to the root chamber, optionally wherein fog supplied to the root chamber is negatively charged; and/or
ii) supplied to the foliage chamber, optionally wherein fog supplied to the foliage chamber is positively charged.

8. The aeroponic propagator (1) of either of claims 6 or 7, further comprising at least one electrically charged device (17), wherein the aeroponic propagator is configured to pass the fog through the at least one electrically charged device (17) to alter the electrical charge of the fog, optionally wherein the aeroponic propagator (1) comprises multiple electrically charged devices which are arranged such that fog being charged passes successively through each of the electrically charged devices, further optionally wherein the aeroponic propagator (1) comprises an elongated structure with a first end and a second end, wherein the fogging system (23) comprises a blowing device (44) configured to move the fog from the first end towards the second end, and wherein the multiple electrically charged devices are arranged along the elongated structure such that fog is charged by each of the electrically charged devices as it is moved along the length of the elongated structure.

9. The aeroponic propagator (1) of any one of claims 6 to 8, wherein the fogging system further comprises at least one blowing device (44) configured to move the fog.

10. The aeroponic propagator (1) of any one of the previous claims, further comprising a reservoir (10) of liquid for use by the droplet generator to generate droplets, wherein the liquid comprises RNA, nitrogen fixing bacteria, and/or a hormone and/or a chemical to promote detachment of produce from cultivated plants.

11. The aeroponic propagator of claim 10, wherein the liquid comprises RNA and further comprising a root chamber (14), in which the roots of plants are disposed in use, and the fog is supplied to the root chamber (14).

12. The aeroponic propagator of either of claim 10 or 11, wherein the liquid comprises RNA and the liquid is lipophilic with a range with a partition coefficient logP_{oktanolwater}between approximately -5 and 5 and more preferably between approximately 0 and 3.

13. A method of supplying fog within an aeroponic propagator (1), the method comprising generating droplets to form the fog using a droplet generator comprising at least one outlet, and **characterised in that** the generation step comprises generating one droplet of liquid at a time from each at least one outlet.

14. The method of claim 13, the method further comprising electrically charging the fog.

15. The method of claim 13 or 14, wherein the liquid comprises RNA.

## Patentansprüche

1. Aeroponischer Vermehrer (1) für die Kultivierung von Pflanzen, wobei der aeroponische Vermehrer (1) umfasst:
ein Vernebelungssystem (23) zur Zuführung von Nebel innerhalb des aeroponischen Vermehrers, wobei das Vernebelungssystem (23) einen Tröpfchengenerator (25) umfasst, wobei der Tröpfchengenerator (25) mindestens einen Auslass (26) umfasst, und **dadurch gekennzeichnet, dass** der Tröpfchengenerator (25) so konfiguriert ist, dass im Gebrauch jeder der mindestens einen Auslässe (26) jeweils ein Flüssigkeitströpfchen ausstößt.

2. Aeroponischer Vermehrer (1) nach Anspruch 1, wobei es mehrere Auslässe gibt und die Auslässe in einer Reihe angeordnet sind.

3. Aeroponischer Vermehrer (1) nach einem der Ansprüche 1 oder 2, wobei der Tröpfchengenerator (25) einen piezoelektrischen Aktuator (29) umfasst, der so konfiguriert ist, dass er den Ausstoß von Tröpfchen aus dem mindestens einen Auslass (26) steuert.

4. Aeroponischer Vermehrer (1) nach einem der vorhergehenden Ansprüche, wobei der Tröpfchengenerator (25) so konfiguriert ist, dass er Folgendes ausstößt:
i) Tröpfchen mit einem Durchmesser von weniger als oder gleich etwa 20 µm; und/oder
ii) etwa mindestens 1000 Tröpfchen pro Sekunde aus jedem der mindestens einen Auslässe.

5. Aeroponischer Vermehrer (1) nach einem der vorhergehenden Ansprüche, wobei der aeroponische Vermehrer eine Wurzelkammer (14) und eine von der Wurzelkammer (14) getrennte Blätterkammer (55) umfasst, und das Vernebelungssystem (23) so konfiguriert ist, dass es die Wurzelkammer und/oder die Blätterkammer mit Nebel versorgt.

6. Aeroponischer Vermehrer (1) nach einem der Ansprüche 1-4, wobei das Vernebelungssystem (23) so konfiguriert ist, dass es im Gebrauch den Nebel elektrisch auflädt.

7. Aeroponischer Vermehrer (1) nach Anspruch 6, wobei der aeroponische Vermehrer eine Wurzelkammer (14) und eine von der Wurzelkammer (14) getrennte Blätterkammer (55) umfasst, und das Vernebelungssystem (23) so konfiguriert ist, dass es der Wurzelkammer und/oder der Blätterkammer Nebel zuführt, wobei das Vernebelungssystem (23) im Gebrauch optional so konfiguriert ist, dass es Nebel elektrisch auflädt, der:
i) der Wurzelkammer zugeführt wird, wobei der der Wurzelkammer zugeführte Nebel optional negativ geladen ist; und/oder
ii) der Blätterkammer zugeführt wird, wobei der der Blätterkammer zugeführte Nebel optional positiv geladen ist.

8. Aeroponischer Vermehrer (1) nach einem der Ansprüche 6 oder 7, der ferner mindestens eine elektrisch geladene Vorrichtung (17) umfasst, wobei der aeroponische Vermehrer so konfiguriert ist, dass er den Nebel durch die mindestens eine elektrisch geladene Vorrichtung (17) leitet, um die elektrische Ladung des Nebels zu verändern, optional, wobei der aeroponische Vermehrer (1) mehrere elektrisch geladene Vorrichtungen umfasst, die so angeordnet sind, dass der aufgeladene Nebel nacheinander durch jede der elektrisch geladenen Vorrichtungen geleitet wird, ferner optional, wobei der aeroponische Vermehrer (1) eine längliche Struktur mit einem ersten Ende und einem zweiten Ende umfasst, wobei das Vernebelungssystem (23) eine Blasvorrichtung (44) umfasst, die so konfiguriert ist, dass sie den Nebel von dem ersten Ende zu dem zweiten Ende bewegt, und wobei die mehreren elektrisch geladenen Vorrichtungen entlang der länglichen Struktur angeordnet sind, so dass der Nebel durch jede der elektrisch geladenen Vorrichtungen aufgeladen wird, wenn er entlang der Länge der länglichen Struktur bewegt wird.

9. Aeroponischer Vermehrer (1) nach einem der Ansprüche 6 bis 8, wobei das Vernebelungssystem ferner mindestens eine Blasvorrichtung (44) umfasst, die zum Bewegen des Nebels konfiguriert ist.

10. Aeroponischer Vermehrer (1) nach einem der vorhergehenden Ansprüche, der ferner ein Flüssigkeitsreservoir (10) zur Verwendung durch den Tröpfchengenerator zur Erzeugung von Tröpfchen umfasst, wobei die Flüssigkeit RNA, stickstofffixierende Bakterien und/oder ein Hormon und/oder eine Chemikalie enthält, um die Ablösung der Produkte von den kultivierten Pflanzen zu fördern.

11. Aeroponischer Vermehrer nach Anspruch 10, wobei die Flüssigkeit RNA enthält und der aeroponische Vermehrer ferner eine Wurzelkammer (14) umfasst, in der die Wurzeln der Pflanzen bei der Verwendung angeordnet sind, und der Nebel der Wurzelkammer (14) zugeführt wird.

12. Aeroponischer Vermehrer nach einem der Ansprüche 10 oder 11, wobei die Flüssigkeit RNA enthält und die Flüssigkeit lipophil ist mit einem Verteilungskoeffizienten logP_{Oktanolwasser} zwischen etwa -5 und 5 und vorzugsweise zwischen etwa 0 und 3.

13. Verfahren zur Zuführung von Nebel in einem aeroponischen Vermehrer (1), wobei das Verfahren die Erzeugung von Tröpfchen zur Bildung des Nebels unter Verwendung eines Tröpfchengenerators mit mindestens einem Auslass umfasst, und **dadurch gekennzeichnet, dass** der Erzeugungsschritt die Erzeugung jeweils eines Flüssigkeitstropfens aus jedem der mindestens einen Auslässe umfasst.

14. Verfahren nach Anspruch 13, wobei das Verfahren ferner das elektrische Aufladen des Nebels umfasst.

15. Verfahren nach Anspruch 13 oder 14, wobei die Flüssigkeit RNA umfasst.

## Revendications

1. Propagateur aéroponique (1) pour la culture de plantes, le propagateur aéroponique (1) comprenant :
un système de brumisation (23) pour fournir un brouillard à l'intérieur du propagateur aéroponique, dans lequel le système de brumisation (23) comprend un générateur de gouttelettes (25), le générateur de gouttelettes (25) comprenant au moins une sortie (26), et **caractérisé en ce que** le générateur de gouttelettes (25) est configuré de telle sorte que, durant l'utilisation, chaque au moins une sortie (26) éjecte une gouttelette de liquide à la fois.

2. Propagateur aéroponique (1) selon la revendication 1, dans lequel il y a de multiples sorties, et les sorties sont agencées en un réseau.

3. Propagateur aéroponique (1) selon l'une ou l'autre des revendications 1 ou 2, dans lequel le générateur de gouttelettes (25) comprend un actionneur piézoélectrique (29) qui est configuré pour commander l'éjection de gouttelettes à partir de l'au moins une sortie (26).

4. Propagateur aéroponique (1) selon l'une quelconque des revendications précédentes, dans lequel le générateur de gouttelettes (25) est configuré pour éjecter :
i) des gouttelettes ayant un diamètre inférieur ou égal à approximativement 20 µm ; et/ou
ii) approximativement au moins 1 000 gouttelettes par seconde à partir de chaque au moins une sortie.

5. Propagateur aéroponique (1) selon l'une quelconque des revendications précédentes, dans lequel le propagateur aéroponique comprend une chambre de racines (14), et une chambre de feuillage (55) séparée de la chambre de racines (14), et le système de brumisation (23) est configuré pour fournir un brouillard à la chambre de racines et/ou la chambre de feuillage.

6. Propagateur aéroponique (1) de l'une quelconque des revendications 1 à 4, dans lequel, durant l'utilisation, le système de brumisation (23) est configuré pour charger le brouillard électriquement.

7. Propagateur aéroponique (1) selon la revendication 6, dans lequel le propagateur aéroponique comprend une chambre de racines (14), et une chambre de feuillage (55) séparée de la chambre de racines (14), et le système de brumisation (23) est configuré pour fournir un brouillard à la chambre de racines et/ou la chambre de feuillage, facultativement dans lequel, durant l'utilisation, le système de brumisation (23) est configuré pour charger le brouillard électriquement :
i) fourni à la chambre de racines, facultativement dans lequel le brouillard fourni à la chambre de racines est chargé négativement ; et/ou
ii) fourni à la chambre de feuillage, facultativement dans lequel le brouillard fourni à la chambre de feuillage est chargé positivement.

8. Propagateur aéroponique (1) selon l'une ou l'autre des revendications 6 ou 7, comprenant en outre au moins un dispositif chargé électriquement (17), dans lequel le propagateur aéroponique est configuré pour faire passer le brouillard à travers l'au moins un dispositif chargé électriquement (17) pour modifier la charge électrique du brouillard, facultativement dans lequel le propagateur aéroponique (1) comprend de multiples dispositifs chargés électriquement qui sont agencés de telle sorte que le brouillard étant chargé passe successivement à travers chacun des dispositifs chargés électriquement, en outre facultativement dans lequel le propagateur aéroponique (1) comprend une structure allongée avec une première extrémité et une seconde extrémité, dans lequel le système de brumisation (23) comprend un dispositif de soufflage (44) configuré pour déplacer le brouillard depuis la première extrémité vers la seconde extrémité, et dans lequel les multiples dispositifs chargés électriquement sont agencés le long de la structure allongée de telle sorte que le brouillard soit chargé par chacun des dispositifs chargés électriquement au fur et à mesure qu'il est déplacé le long de la longueur de la structure allongée.

9. Propagateur aéroponique (1) selon l'une quelconque des revendications 6 à 8, dans lequel le système de brumisation comprend en outre au moins un dispositif de soufflage (44) configuré pour déplacer le brouillard.

10. Propagateur aéroponique (1) selon l'une quelconque des revendications précédentes, comprenant en outre un réservoir (10) de liquide destiné à être utilisé par le générateur de gouttelettes pour générer des gouttelettes, dans lequel le liquide comprend ARN, des bactéries fixatrices d'azote, et/ou une hormone et/ou un produit chimique pour favoriser la séparation de produits à partir de plantes cultivées.

11. Propagateur aéroponique selon la revendication 10, dans lequel le liquide comprend ARN, et comprenant en outre une chambre de racines (14), dans lequel les racines de plantes sont disposées, durant l'utilisation, et le brouillard est fourni à la chambre de racines (14).

12. Propagateur aéroponique selon l'une ou l'autre de la revendication 10 ou 11, dans lequel le liquide comprend ARN et le liquide est lipophile avec une plage avec un coefficient de partage logP_{oktanolwater} entre approximativement -5 et 5 et de façon davantage préférée entre approximativement 0 et 3.

13. Procédé de fourniture de brouillard à l'intérieur d'un propagateur aéroponique (1), le procédé comprenant la génération de gouttelettes pour former le brouillard en utilisant un générateur de gouttelettes comprenant au moins une sortie, et **caractérisé en ce que** l'étape de génération comprend la génération d'une gouttelette de liquide à la fois à partir de chaque au moins une sortie.

14. Procédé selon la revendication 13, le procédé comprenant en outre le chargement électrique du brouillard.

15. Procédé selon la revendication 13 ou 14, dans lequel le liquide comprend ARN.
